# EUROPEAN PATENT SPECIFICATION

(11) **EP 0 683 787 B1**
(45) Date of publication and mention of the grant of the patent: **10.09.1997**
(21) Application number: 95903277.2
(22) Date of filing: 24.11.1994
(51) Int. Cl.: C07H 15/252, A61K 31/70

(54) **4'-O-SULFONYL-ANTHRACYCLINE DERIVATIVES**
4'-0-SULFONYLANTHRACYCLINDERIVATE
DERIVES DE 4'-O-SULFONYL-ANTHRACYCLINE

(30) Priority: 13.12.1993 GB 9325420
(43) Date of publication of application: 29.11.1995
(73) Proprietor: PHARMACIA & UPJOHN S.p.A., 20152 Milano (IT)
(72) Inventor: BARGIOTTI, Alberto, I-20146 Milan (IT); CARUSO, Michele, I-20131 Milan (IT); GRANDI, Maria, I-20131 Milan (IT); RIPAMONTI, Marina, I-20131 Milan (IT); SUARATO, Antonino, I-20158 Milan (IT)
(74) Representative: Giambrocono, Alfonso, Dr. Ing.
(86) International application number: EP9403893
(87) International publication number: WO9516693

(56) References cited:
- WO-A-93/01201
- BE-A- 902 610

## Description

The invention relates to new anthracycline glycosides, to a process for their preparation and to pharmaceutical compositions containing them.

A new class of anthracycline glycoside antibiotics in which the 4'-hydroxy group of the sugar residue is substituted with a sulfonate has now been found. The present invention therefore provides a compound which is an anthracycline glycoside of formula 1 or 2 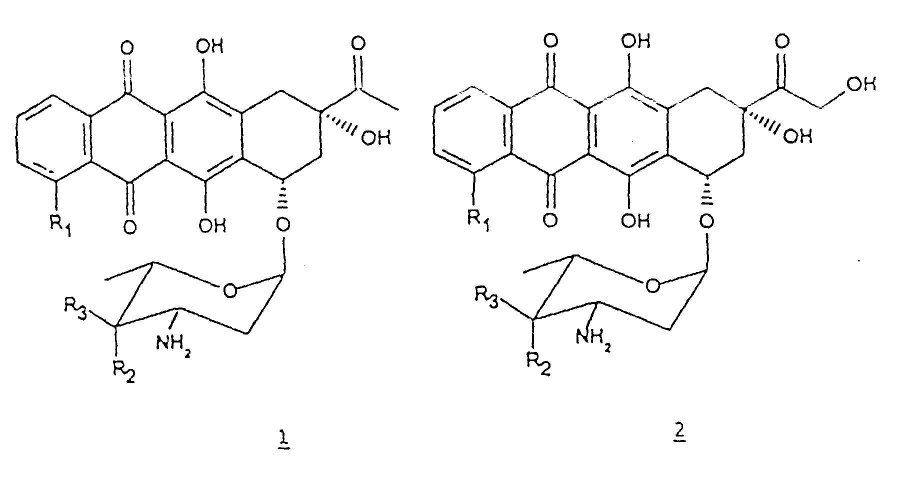 wherein R₁ is hydrogen or a methoxy group; one of R₂ and R₃ is hydrogen and the other is a sulfonyl residue R₄SO₂O- in which R₄ is a methyl, ethyl, n-propyl or isopropyl; or a pharmaceutically acceptable salt thereof.

Halogen in the present text means fluorine, chlorine, bromine or iodine, preferably fluorine or chlorine, more preferably chlorine.

Particularly preferred salts of the compounds of the present invention are pharmaceutically acceptable addition salts such as hydrochlorides.

Particularly preferred compounds of formula 1 include:
(1a) 4'-O-methansulfonyldaunorubicin (R₁=OCH₃, R₂=CH₃SO₂O, R₃=H)
(1b) 4-demethoxy-4'-O-methansulfonyldaunorubicin (R₁=R₃=H, R₂=CH₃SO₂O)
(1c) 4'-epi-4'-O-methansulfonyldaunorubicin (R₁=OCH₃, R₂=H, R₃=CH₃SO₂O)

Particularly preferred compounds of formula 2 include:
(2a) 4'-O-methansulfonyldoxorubicin (R₁=OCH₃, R₃=H, R₂=CH₃SO₂O)
(2b) 4-demethoxy-4'-O-methansulfonyldoxorubicin (R₁=R₃=H, R₂=CH₃SO₂O)
(1c) 4'-epi-4'-O-methansulfonyldoxorubicin (R₁=OCH₃, R₂=H, R₃=CH₃SO₂O)

Accordingly, the present invention provides a process for the preparation of a compound which is an anthracycline glycoside of formula 1 or 2 as above defined or a pharmaceutically acceptable salt thereof, which process comprises:
(a) reacting an anthracycline of general formula 3 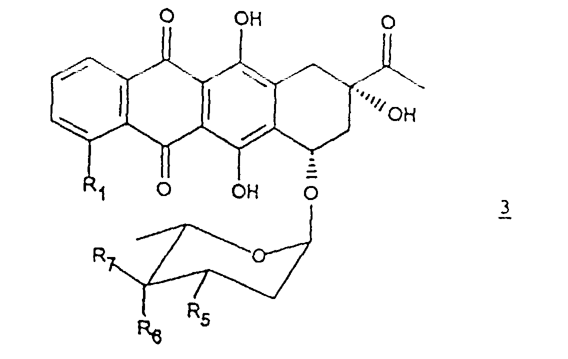 wherein, R₁ has the same meaning as above defined, R₅ represents a protected amino group, one of R₆ and R₇ is hydrogen and the other is hydroxy, with a substituted sulfonyl halogen derivative of formula 4

   R₄SO₂-X 4

   wherein R₄ has the same meaning as defined above and X is halogen, preferably chlorine, in the presence of a weak organic base; and
(b) isolating the N-protected derivative of formula 5 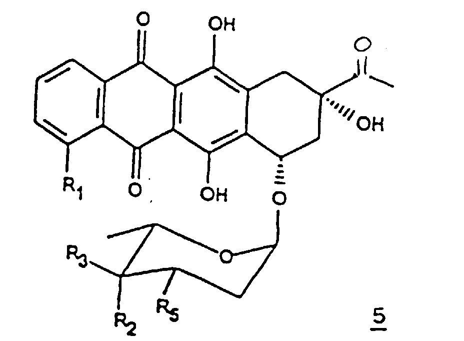 wherein R₁, R₂, R₃ and R₅ are as above defined and preferably purifying said derivative e.g. by column chromatography;
(c) deblocking the N-protected amino group of said derivative 5 in the presence of a base preferably at from 0°C to 5°C and for 4 to 8 hours; and if desired, converting the compound of formula 1 thus obtained into a pharmaceutically acceptable salt thereof; or
(d) converting a compound of formula 1 into the corresponding derivative of formula 2 by hydroxylation at the C-14 position e.g. following the procedure described in US Patent No: 3,803,124 and, if desired, converting the compound of formula 2 thus obtained into a pharmaceutically acceptable salt thereof.

It should be noted that use of weak organic base in step (a) avoids sulfonylation at phenolic hydroxyl.

As examples of starting anthracyclines of general formula 3 there may be given: N-trifluoroacetyldaunorubicin (3a: R₁=OCH₃, R₅=NHCOCF₃, R₆=OH, R₇=H), 4-demethoxy-N-trifluoroacetyldaunourubicin (3b: R₁=R₇=H, R₅=NHCOCF₃, R₆=OH), 4'-epi-N-trifluoroacetyldaunourubicin (3c: R₁=OCH₃, R₅=NHCOCF₃, R₆=H, R₇=OH). Said derivatives may be prepared as described by F. Arcamone in "DOXORUBICIN" Medicinal Chemistry, Vol. 17, Academic Press INC (London) 1981. Other compounds of formula 3 may be prepared by analogous methods.

The O-sulfonylation of compound 3 is typically carried out using an excess, e.g. from 1.2 to 20 equivalents preferably 1.2 to 5 equivalents, of halo sulfonyl derivative 4. The weak organic base present in step (a) may be provided by carrying out the reaction in an appropriate medium. For example, depending on the reactivity of the sulfonylating agent, the reaction may be carried out in anhydrous pyridine and can usually be completed from 15 to 60 hours, at a temperature from 0°C to room temperature, preferably at about 4°C. Alternatively, the reaction may be carried out in an organic solvent, such as dry methylene chloride, in the presence of an organic base e.g. pyridine or collidine. The reaction can usually be completed from 15 to 60 hours, at a temperature from 0°C to room temperature, preferably at about 4°C. The reaction product is isolated, as N-trifluoroacetate derivative (5) from the reaction mixture e.g. by solvent extraction and preferably purified e.g. by column chromatography. In order to deblock the N-protected group, the N-trifluoroacetate-4'-O-sulfonyl derivative is subjected to basic conditions e.g. contacting with aqueous 0.1 to 0.5N sodium -hydroxide, at a temperature from 0°C to 5°C, for a time from 4 to 8 hours. The product thus obtained may be extracted from the reaction mixture e.g. with methylene chloride, the organic solvent concentrated to small volume, and the desired product isolated as its hydrochloride by treatment with methanolic hydrogen chloride.

In order to prepare sulfonated anthracycline glycosides of formula 2, compounds 1 are hydroxylated at C-14 for example as described in US Patent No: 3,803,124. The products may be obtained as their hydrochloride by treatment with methanolic hydrogen chloride.

As a further aspect, the invention provides pharmaceutical compositions comprising an anthracycline glycoside of formula 1 or 2 or a pharmaceutically acceptable salt thereof in combination with a pharmaceutically acceptable diluent or carrier. Conventional carriers and diluents may be used. The compositions may be formulated and administered in a conventional manner.

Suitable routes of administration include parenteral administration. For parenteral administration a liquid formulation may be prepared using the active compound and a sterile diluent or carrier which may either dissolve the active compound or provide a suspension for it. The parenteral formulation may be prepared in the form of a sterile solid for reconstitution prior to administration with a suitable vehicle such as physiological saline, sterile water or other sterile vehicle.

The compounds of the invention are useful in methods of treatment of the human and animal body by therapy. They are useful as anti-tumor agents in particular in the treatment of leukaemia or colon adenocarcinoma. A therapeutically effective amount is administered to a patient having a tumor to ameliorate or improve the condition of the patient. An amount sufficient to inhibit the growth of the tumor may be administered. The dosage to be given can be ascertained using known dosage ranges for doxorubicin and daunorubicin modified by reference to the activity shown by the present compounds in *in vitro* and *in vivo* anti-tumor tests. Suitable dosages are generally in the range of 1 to 200 mg/m² body surface preferably from 1 to 100 mg/m² depending on the nature and severity of the disease being treated and on the general condition of the patient.

The following examples illustrate the invention.

### EXAMPLE 1:

### Preparation of 4'-O-methansulfonyldaunorubicin (1a: R₁=OCH₃, R₂=CH₃SO₂O, R₃=H)

N-trifluoroacetyldaunorubicin (3a: 5g, 8.2 mmol) was dissolved in dry pyridine (30 ml) and added to methanesulfonyl chloride (0.9 ml, 11.5 mmol) at 0°C. The reaction mixture was stirred for 1.5 hours, and subsequently poured onto ice. The precipitate was collected, washed with water and dried to give 4'-O-methansulfonyl-N-trifluoroacetyldaunorubicin (5a, 5.6 g). TLC plate on Kieselgel F258 (Merck), with an eluting system of methylene chloride/methanol (20:1 by volume) Rf=0.57.

Compound 5a (4g) was added to aqueous 0.3N sodium hydroxide (200 ml) and stirred at 10-15°C for 6 hours. The reaction mixture was then brought to pH 3 with 1N hydrochloric acid and extracted with methylene chloride. The aqueous solution was brought to pH 7.5 and extracted with methylene chloride (3x100 ml). The organic phase was washed with water, dried over anhydrous sodium sulfate, concentrated under reduced pressure and treated with a solution of methanolic anhydrous hydrogen chloride to give, after precipitation with ethyl ether, the title compound la (2.6 g). TLC plate on Kieselgel F258 (Merck), eluting system methylene chloride/methanol (10:1 by volume) Rf=0.35.
FD-MS: m/z 605 (M+)

### EXAMPLE 2

### Preparation of 4-demethoxy-4'-O-methansulfonyldaunorubicin (1b: R₁=R₃=H, R₂=CH₃SO₂O)

The title compound 1b was prepared starting from 4-demethoxy-N-trifluoroacetyldaunorubicin (3b: 5.7 g, 10 mmol) following the same procedure described in Example 1. Yield of 1b: 3 g.

TLC plate on Kieselgel F258 (Merck), eluting system methylene chloride/methanol (10:1 by volume)
Rf=0.5.
FD-MS: m/z 575 (M+)

### EXAMPLE 3

### Preparation of 4'-O-methansulfonyldoxorubicin

### (2a: R₁=OCH₃, R₄=H, R₃=CH₃SO₂O)

4'-O-methansufonyldaunorubicin hydrochloride (1a: 0.6 g, 1 mmol) prepared as described in Example 1 was converted into the title compound 2a as described in US Patent No: 3,803,124 (yield 0.4 g). TLC plate on Kieselgel F258 (Merck), eluting system methylene chloride/methanol (10:1 by volume) Rf=0.21; m.p. 157-158°C; FD-MS: m/z 621 (M+).

### Biological activity

4'-O-methansufonyl derivatives 1b and 2a were tested *in vitro* on two human cell lines, LoVo (colon adenocarcinoma) and LoVo/DX (colon adenocarcinoma resistant to doxorubicin) in comparison with doxorubicin. The cytotoxic activity is reported as IC50, the concentration inhibiting 50% of colony formation, calculated on concentration response curves. Resistance index R.I. is the ratio between the IC50 on resistant cells and the IC50 on sensitive cells. Both compounds were found more cytotoxic than doxorubicin (Table I).

Compounds 1b and 2a were also evaluated *in vivo* against P388 murine leukaemia resistant to doxorubicin (10⁵ cell/mouse transplanted i.v. in BD2F1 mice) in comparison with doxorubicin (Table II).

The introduction of a sulfonyl residue at position C-4' of anthracycline produces a new class of anthracyclines endowed with activity against doxorubicin-resistant tumors.

**Table I:**

| *in vivo* cytotoxic activity (IC50) of compound 1b and 2a on LoVo and LoVo/DX cells in comparison with doxorubicin. | | | |
|---|---|---|---|
| **IC50 = (ng/ml)** ^{**(1)**} | | | |
| Compound | LoVo | LoVo/DX | R.I. ⁽²⁾ |
| 1b | 10.5 | 60.5 | 5.8 |
| 2a | 17.3 | 1800.0 | 104.0 |
| doxorubicin | 49.0 | 2554.0 | 52.1 |

| | | | |
|---|---|---|---|
| Colony assay: 4 h treatment ⁽¹⁾ IC50 = concentration inhibiting 50% colony formation; | | | |
| ⁽²⁾ R.I. = Resistance Index = (IC50 Lovo/DX)/(IC50 LoVo). | | | |

**Table II:**

| Antitumor activity of 1b and 2a on P388/DX leukaemia in comparison with doxorubicin. | | |
|---|---|---|
| Compound | O.D.⁽¹⁾ mg/kg | T/C⁽²⁾ % |
| 1b | 8.1 | 133 |
| 2a | 40.0 | 137 |
| doxorubicin | 16.9 | 106 |

| | | |
|---|---|---|
| The compounds were injected i.v. one day after tumor transplantation. ⁽¹⁾ Optimal Dose | | |
| ⁽²⁾ Median survival time of treated mice/Median survival time of controls x 100. | | |

## Claims

1. A compound which is an anthracycline glycoside of general formula 1 or 2: 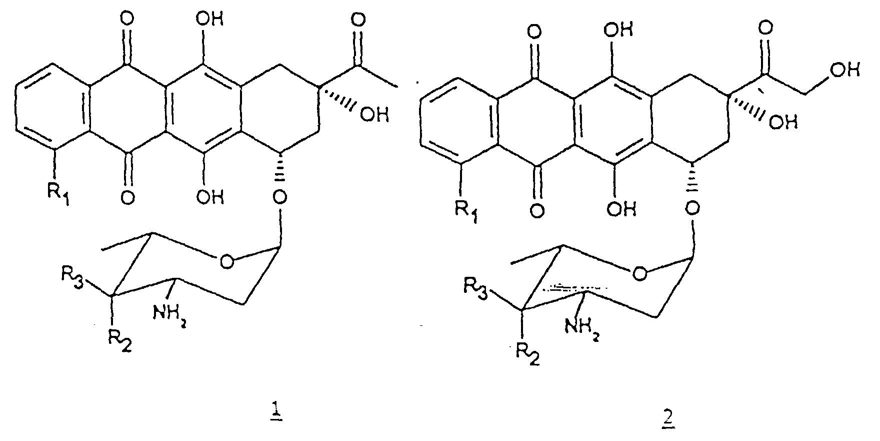 wherein R₁ is hydrogen or a methoxy group; one of R₂ and R₃ is hydrogen and the other is a sulfonyl residue R₄SO₂O- in which R₄ is a methyl, ethyl, n-propyl or isopropyl; or a pharmaceutically acceptable salt thereof.

2. A compound according to claim 1, which is:
(1a) 4'-O-methansulfonyldaunorubicin (R₁=OCH₃, R₂=CH₃SO₂O, R₃=H)
(1b) 4-demethoxy-4'-O-methansulfonyldaunorubicin (R₁=R₃=H, R₂=CH₃SO₂O)
(1c) 4'-epi-4'-O-methansulfonyldaunorubicin (R₁=OCH₃, R₂=H, R₃=CH₃SO₂O)
(2a) 4'-O-methansulfonyldoxorubicin (R₁=OCH₃, R₃=H, R₂=CH₃SO₂O)
(2b) 4-demethoxy-4'-O-methansulfonyldoxorubicin (R₁=R₃=H, R₂=CH₃SO₂O)
(2c) 4'-epi-4'-O-methansulfonyldoxorubicin (R₁=OCH₃, R₂=H, R₃=CH₃SO₂O),
or a pharmaceutically acceptable salt thereof.

3. A compound according to any one of claims 1 to 2, which is in the form of a hydrochloride salt.

4. A process for the preparation of a compound as defined in any one of claims 1 to 3, which process comprises:
(a) reacting an anthracycline of general formula 3 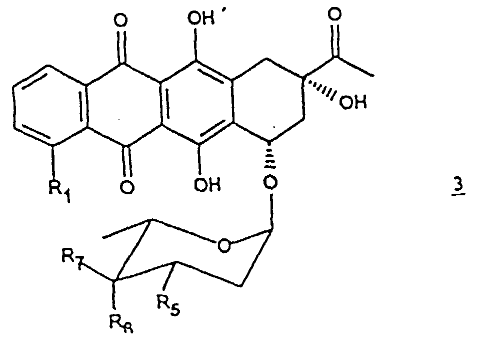 wherein R₁ is as defined in claim 1, R₅ represents a protected amino group, one of R₆ and R₇ is hydrogen and the other is hydroxy, with a substituted sulfonyl halogen derivative of formula 4
R₄SO₂-X 4
wherein R₄ is as defined in claim 1 and X is halogen, in the presence of a weak organic base;
(b) isolating the N-protected derivative of formula 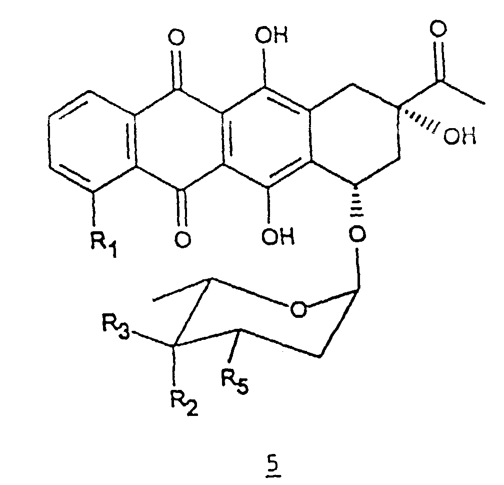 wherein R₁, R₂, R₃ and R₅ are as defined in claim 1;
(c) deblocking the N-protected amino group of said derivative 5 in the presence of a base; and if desired, converting the compound of formula 1 thus obtained into a pharmaceutically acceptable salt thereof; or
(d) converting a compound of formula 1 by hydroxylation into the corresponding derivative of formula 2 and, if desired, converting the compound of formula 2 thus obtained into a pharmaceutically acceptable salt thereof.

5. A process according to claim 4, wherein the halogen is chlorine.

6. A process according to claim 4 or 5, wherein the organic base used in step (a) is selected from the group of pyridine and collidine.

7. A process according to claim 4, 5 or 6, wherein the base used in step (c) is sodium hydroxide.

8. A process according to any one of claims 4 to 7, wherein step (a) is carried out in an organic solvent at from 0°C to room temperature for 15 to 60 hours, the purification in step (b) is carried out using column chromatography and step (c) is carried out at from 0°C to 5°C for 4 to 8 hours.

9. A process according to claim 8, wherein the organic solvent is pyridine or methylene chloride.

10. A pharmaceutical composition comprising a pharmaceutically acceptable diluent or carrier and, as an active principle, a compound as defined in any one of claims 1 to 3.

11. An anthracycline glycoside of formula 1 or 2 or a pharmaceutically acceptable salt thereof, as defined in claim 1, for use in a method of treatment of the human or animal body by therapy.

12. An anthracycline glycoside or a pharmaceutically acceptable salt thereof as defined in claim 1, for use as an anti-tumor agent.

## Patentansprüche

1. Verbindung, die ein Anthracyclinglycosid der allgemeinen Formel 1 oder 2 ist: 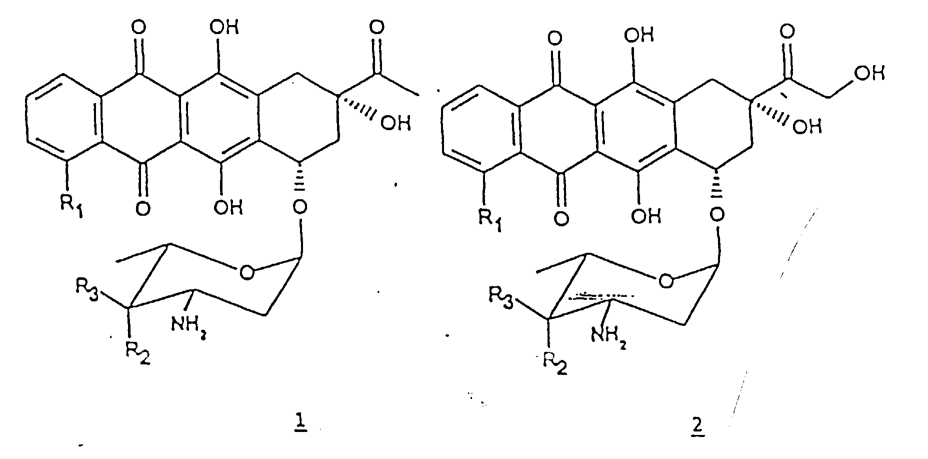 worin R₁ Wasserstoff oder eine Methoxygruppe ist; eines von R₂ und R₃ Wasserstoff und das andere ein Sulfonylrest R₄SO₂O- ist, worin R₄ ein Methyl, Ethyl, n-Propyl oder Isopropyl ist; oder ein pharmazeutisch akzeptables Salz davon.

2. Verbindung nach Anspruch 1, die ist:
(1a) 4'-O-Methansulfonyldaunorubicin (R₁=OCH₃, R₂ = CH₃SO₂O, R₃ = H)
(1b) 4-Demethoxy-4'-O-methansulfonyldaunorubicin (R₁ =R₃=H, R₂=CH₃SO₂O)
(1c) 4'-Epi-4'-O-methansulfonyldaunorubicin (R₁ = OCH₃, R₂=H, R₃ =CH₃SO₂O)
(2a) 4'-O-Methansulfonyldoxorubicin (R₁=OCH₃, R₃=H, R₂=CH₃SO₂O)
(2b) 4-Demethoxy-4'-O-methansulfonyldoxorubicin (R₁ =R₃=H, R₂=CH₃SO₂O)
(2c) 4'-Epi-4'-O-methansulfonyldoxorubicin (R₁ = OCH₃, R₂=H, R₃ =CH₃SO₂O)
oder ein pharmazeutisch akzeptables Salz davon.

3. Verbindung nach einem der Ansprüche 1 bis 2, die in der Form eines Hydrochloridsalzes vorliegt.

4. Verfahren zur Herstellung einer Verbindung, wie in einem der Ansprüche 1 bis 3 definiert, umfassend:
(a) Reaktion eines Anthracyclins der allgemeinen Formel 3 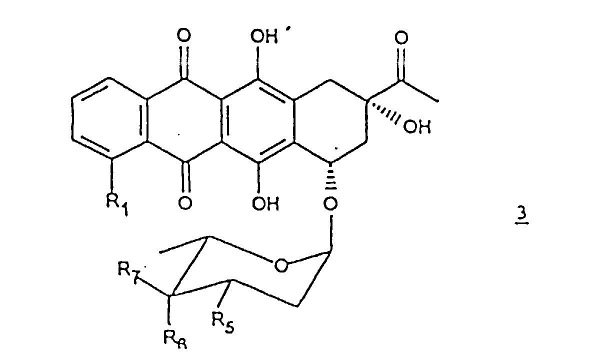 worin R₁ wie in Anspruch 1 definiert ist, R₅ eine geschützte Aminogruppe ist, eines von R₆ und R₇ Wasserstoff und das andere Hydroxy ist, mit einem substituierten sulfonylhaltigen Derivat der Formel 4
R₄SO₂-X 4
worin R₄ wie in Anspruch 1 definiert ist und X Halogen ist, in der Gegenwart einer schwachen organischen Base; und
(b) Isolation des N-geschützten Derivates der Formel 5 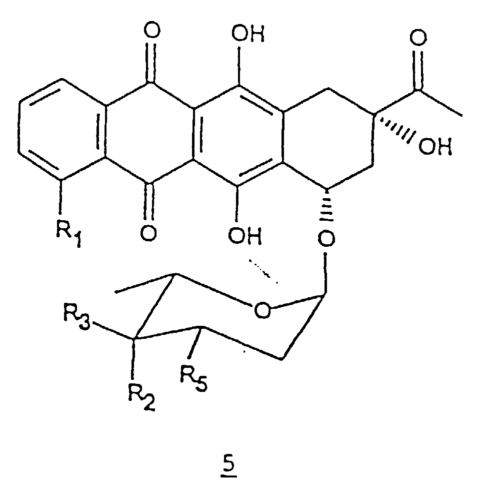 worin R₁, R₂, R₃ und R₅ wie in Anspruch 1 definiert sind;
(c) Abspalten der Schutzgruppe der N-geschützten Aminogruppe des Derivates 5 in der Gegenwart einer Base, und falls gewünscht, Umwandlung der somit erhaltenen Verbindung der Formel (1) in ein pharmazeutisch akzeptables Salz davon; oder
(d) Umwandlung einer Verbindung der Formel (1) in das entsprechende Derivat der Formel (2) durch Hydroxylierung in das entsprechende Derivat der Formel 2 und, falls gewünscht, Umwandlung der somit erhaltenen Verbindung der Formel 2 in ein pharmazeutisch akzeptables Salz davon.

5. Verfahren nach Anspruch 4, worin das Halogen Chlor ist.

6. Verfahren nach Anspruch 4 oder 5, worin die in Schritt (a) verwendete organische Base ausgewählt wird aus der Gruppe aus Pyridin und Collidin.

7. Verfahren nach den Ansprüchen 4, 5 oder 6, worin die in Schritt (c) verwendete Base Natriumhydroxid ist.

8. Verfahren nach einem der Ansprüche 4 bis 7, worin der Schritt (a) in einem organischen Lösungsmittel bei 0°C bis Raumtemperatur für 15 bis 60 Stunden, die Reinigung in Schritt (b) unter Anwendung der Säulenchromatographie und der Schrit (c) bei 0 bis 5°C für 4 bis 8 h durchgeführt werden.

9. Verfahren nach Anspruch 8, worin das organische Lösungsmittel Pyridin oder Methylenchlorid ist.

10. Pharmazeutische Zusammensetzung, umfassend ein pharmazeutisch akzeptables Verdünnungsmittel oder Träger, und als einen aktiven Bestandteil eine Verbindung, wie in einem der Ansprüche 1 bis 3 definiert.

11. Anthracyclinglycosid der Formel 1 oder 2 oder ein pharmazeutisch akzeptables Salz davon, wie in Anspruch 1 definiert, zur Verwendung in einem Verfahren zur Behandlung des menschlichen oder tierischen Körpers durch Therapie.

12. Anthracyclinglycosid oder ein pharmazeutisch akzeptables Salz davon, wie in Anspruch 1 definiert, zur Verwendung als ein Antitumormittel.

## Revendications

1. Composé qui est un anthracycline glycoside de formule générale 1 ou 2 : 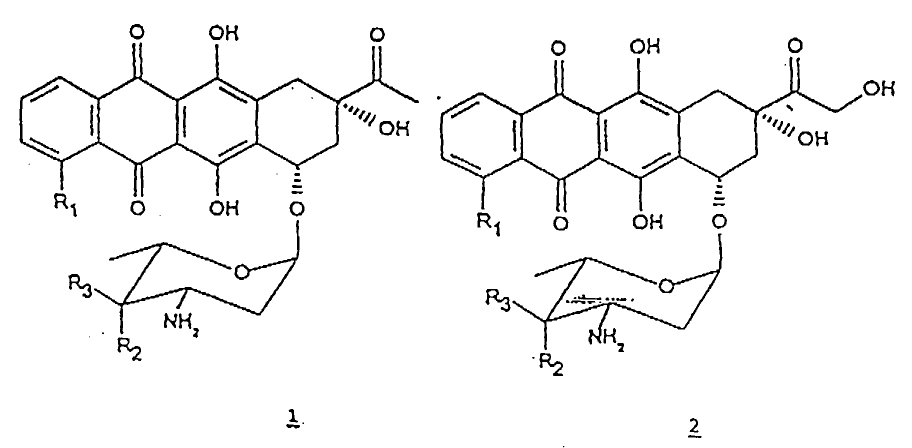 où :
- R₁ représente hydrogène ou un groupe méthoxy ;
- l'un parmi R₂ et R₃ représente hydrogène, et l'autre est un reste sulfonyle R₄SO₂O- dans lequel R₄ est un groupe méthyle, éthyle, n-propyle ou isopropyle ;
ou un sel pharmaceutiquement acceptable de ce composé.

2. Composé selon la revendication 1, qui est :
(1a) la 4'-O-méthanesulfonyldaunorubicine (R₁=OCH₃, R₂=CH₃SO₂O, R₃=H) ;
(1b) la 4-déméthoxy-4'-O-méthanesulfonyldaunorubicine (R₁=R₃=H, R₂=CH₃SO₂O) ;
(1c) la 4'-épi-4'-O-méthanesulfonyldaunorubicine (R₁=OCH₃, R₂=H, R₃=CH₃SO₂O) ;
(2a) la 4'-O-méthanesulfonyldoxorubicine (R₁=OCH₃, R₃=H, R₂=CH₃SO₂O) ;
(2b) la 4-déméthoxy-4'-O-méthanesulfonyldoxorubicine (R₁=R₃=H, R₂=CH₃SO₂O) ;
(2c) la 4'-épi-4'-O-méthanesulfonyldoxorubicine (R₁=OCH₃, R₂=H, R₃=CH₃SO₂O),
ou un sel pharmaceutiquement acceptable de ces composés.

3. Composé selon l'une quelconque des revendications 1 et 2, qui se présente sous la forme d'un sel chlorhydrate.

4. Procédé de préparation d'un composé tel que défini à l'une des revendications 1 à 3, lequel procédé comprend :
(a) la réaction d'une anthracycline de formule générale 3 : 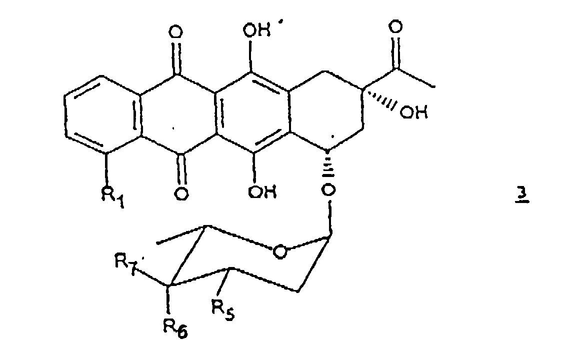 dans laquelle :
- R₁ est tel que défini à la revendication 1 ;
- R₅ représente un groupe amino protégé ;
- l'un parmi R₆ et R₇ représente hydrogène et l'autre représente hydroxy,
avec un dérivé halogénure de sulfonyle substitué de formule 4 :
R₄SO₂-X 4
dans laquelle :
- R₄ est tel que défini à la revendication 1 ; et
- X représente halogène,
en présence d'une base organique faible ;
(b) l'isolement du dérivé N-protégé de formule : 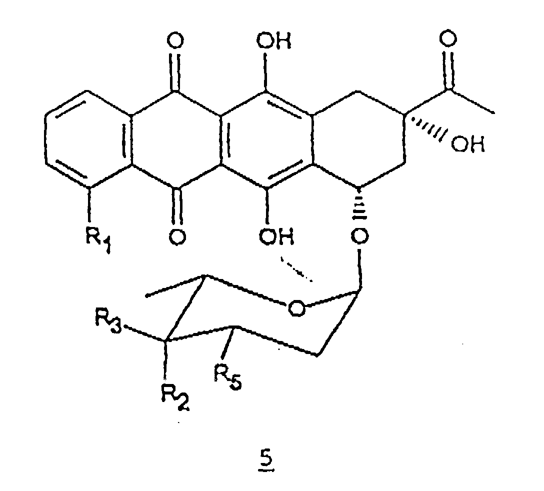 dans laquelle R₁, R₂, R₃ et R₅ sont tels que définis à la revendication 1 ;
(c) le déblocage du groupe amino N-protégé dudit dérivé 5 en présence d'une base ; et, si on le désire, la conversion du composé de formule 1 ainsi obtenu en un sel pharmaceutiquement acceptable de celui-ci ; ou
(d) la conversion d'un composé de formule 1 par hydroxylation en le dérivé correspondant de formule 2 et, si on le désire, la conversion du composé de formule 2 ainsi obtenu en un sel pharmaceutiquement acceptable de celui-ci.

5. Procédé selon la revendication 4, dans lequel l'halogène est le chlore.

6. Procédé selon la revendication 4 ou 5, dans lequel la base organique utilisée à l'étape (a) est choisie dans le groupe de la pyridine et de la collidine.

7. Procédé selon la revendication 4, 5 ou 6, dans lequel la base utilisée à l'étape (c) est l'hydroxyde de sodium.

8. Procédé selon l'une quelconque des revendications 4 à 7, dans lequel l'étape (a) est effectuée dans un solvant organique à une température de 0°C à la température ambiante pendant 15 à 60 heures, la purification à l'étape (b) est effectuée par chromatographie sur colonne, et l'étape (c) est effectuée à une température de 0°C à 5°C pendant 4 à 8 heures.

9. Procédé selon la revendication 8, dans lequel le solvant organique est la pyridine ou le chlorure de méthylène.

10. Composition pharmaceutique comprenant un diluant ou support pharmaceutiquement acceptable et, comme principe actif, un composé tel que défini à l'une quelconque des revendications 1 à 3.

11. Anthracycline glycoside de formule 1 ou 2 ou un sel pharmaceutiquement acceptable de celui-ci, tel que défini à la revendication 1, pour utilisation dans un procédé de traitement du corps humain ou animal par thérapie.

12. Anthracycline glycoside ou sel pharmaceutiquement acceptable de celui-ci, tel que défini à la revendication 1, pour utilisation en tant qu'agent anti-tumoral.
